(19) Europäisches Patentamt
European Patent Office
Office européen des brevets

(11) Veröffentlichungsnummer: **0 252 299**
**A2**

(12) # EUROPÄISCHE PATENTANMELDUNG

(21) Anmeldenummer: 87108094.1

(22) Anmeldetag: 04.06.87

(30) Priorität: 06.06.86 DE 3619099

(43) Veröffentlichungstag der Anmeldung:
13.01.88 Patentblatt 88/02

(84) Benannte Vertragsstaaten:
AT BE CH DE ES FR GB GR IT LI LU NL SE

(51) Int. Cl.⁴: A61K 31/50

(71) Anmelder: BOEHRINGER INGELHEIM KG

D-6507 Ingelheim am Rhein(DE)

(84) AT BE CH DE ES FR GB GR IT LI LU NL SE

(71) Anmelder: Boehringer Ingelheim International G.m.b.H

D-6507 Ingelheim am Rhein(DE)

(84) GB

(72) Erfinder: Lösel, Walter, Dr.
Im Herzenacker 26
D-6535 Gau-Algesheim(DE)
Erfinder: Roos, Otto, Dr.
Elsheimer Strasse 36
D-6501 Schwabenheim(DE)
Erfinder: Schnorrenberg, Gerd, Dr.
Ernst-Ludwig-Strasse 66a
D-6535 Gau-Algesheim(DE)
Erfinder: Arndts, Dietrich, Dr.
Mühlstrasse 7
D-6531 Appenheim(DE)
Erfinder: Speck, Georg,Dr.
Rheinstrasse 13
D-6507 Ingelheim am Rhein(DE)
Erfinder: Ensinger, Helmut, Dr.
Im Herrengarten 10
D-6501 Wackernheim(DE)
Erfinder: Kuhn, Franz-Josef, Dr.
Beethovenstrasse 11
D-6535 Gau-Algesheim(DE)
Erfinder: Lehr, Erich, Dr.
In der Toffel 5
D-6531 Waldalgesheim(DE)
Erfinder: Hinzen, Dieter, Prof. Dr.
Konrad-Adenauer-Strasse 26
D-6501 Zornheim(DE)
Erfinder: Schingnitz, Günter, Dr.
Unter den Gärten 55
D-6535 Gau-Algesheim(DE)
Erfinder: Reichl, Richard, Dr.
Im Hippel 55
D-6535 Gau-Algesheim(DE)

EP 0 252 299 A2

Xerox Copy Centre

Erfinder: **Streller, Ilse, Dr.**
**Waldstrasse 16**
**D-6534 Stromberg(DE)**

(54) **Verwendung von 12-Amino-pyridazino [4',5' : 3,4] pyrrolo[2,1-a] isochinolinen zur Herstellung von cardio- und neuroprotektiven Mitteln.**

(57) Die Erfindung betrifft die Verwendung von 12-Amino-pyridazino-[4',5' : 3,4] pyrrolo [2,1-a] isochinolinen der Formel

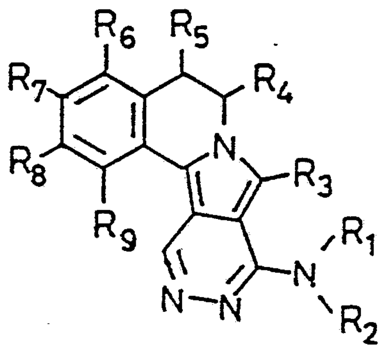

(I)

sowie deren physiologisch verträglichen Salze mit Säuren, Basen und Komplexbildnern zur Herstellung von cardio-und/oder neuroprotektiven Mitteln.

# Verwendung von 12-Amino-pyrridazino[4',5' : 3,4]pyrrolo-[2,1-a] isochinolinen zur Herstellung von cardio-und neuroprotektiven Mitteln

Die Erfindung betrifft die Verwendung von 12-Amino-pyridazino [4',5' : 3,4] pyrrolo [2,1-a] isochinolinen der Formel

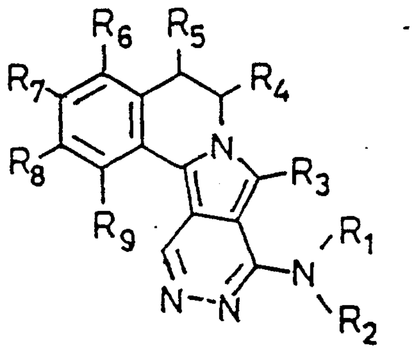

(I)

sowie deren physiologisch verträgliche Salze mit Säuren, Basen und Komplexbildnern zur Herstellung von cardio-und neuroprotektiven Mitteln.

In Formel I bedeuten:

$R_1$ und $R_2$, die gleich oder verschieden sein können, Wasserstoff; $C_3$-$C_7$ Cycloalkyl; $C_2$-$C_5$ Alkenyl; Phenyl (wobei der Phenylring gegebenenfalls ein-oder zweifach durch Halogen oder Methoxy substituiert ist); Propargyl; einen geradkettigen oder verzweigten, gesättigten oder ungesättigten Alkylrest mit 1 - 5 Kohlenstoffatomen, der substituiert sein kann durch Hydroxy, Alkoxy mit 1 - 4 Kohlenstoffatomen, Halogen, $NH_2$, NH-Alkyl mit 1 - 2 Kohlenstoffatomen, N,N-Dialkyl mit 1 - 2 Kohlenstoffatomen, NH-Acyl mit 2 - 4 Kohlenstoffatomen, Cycloalkyl mit 3 - 7 Kohlenstoffatomen, Phenyl (wobei der Phenylring seinerseits substituiert sein kann ein-oder mehrfach durch Halogen, Alkyl mit 1 - 2 Kohlenstoffatomen, Alkoxy mit 1 - 2 Kohlenstoffatomen, NH-Alkyl mit 1 - 2 Kohlenstoffatomen, N,N-Dialkyl mit 1 - 2 Kohlenstoffatomen, $NH_2$, N-Acyl mit 2 - 3 Kohlenstoffatomen oder Alkylsulfonylamino), Furyl, Thienyl, einen stickstoffhaltigen heterocyclischen 5-oder 6-Ring der gegebenenfalls als weiteres Heteroatom ein Sauerstoff-oder Schwefelatom enthalten kann (wobei der Ring gegebenenfalls durch Alkyl mit 1 - 4 Kohlenstoffatome substituiert ist);

$R_1$ und $R_2$ zusammen mit dem Stickstoffatom einen 3-bis 7-gliedrigen Ring, der gegebenenfalls als weiteres Heteroatom ein Sauerstoff-oder ein Stickstoffatom enthalten kann (wobei der Ring gegebenenfalls durch Phenyl-($C_1$-$C_4$)-Alkyl substituiert ist (wobei der Phenylring seinerseits ein-oder zweifach durch Halogen oder Methoxy substituiert ist));

$R_2$, falls $R_1$ Wasserstoff bedeutet, einen $-NH_2$; Di($C_1$-$C_2$) Alkylamino; Acetonylamino; -NH($C_2$-$C_3$)Acyl; Alkylsulfonyl-oder Alkoxycarbonylrest mit je 1 - 3 Kohlenstoffatomen in der Alkylkette; die Isopropylidenaminogruppe

( 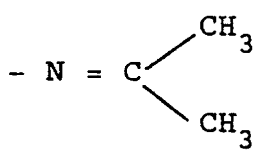 )

oder einen heterocyclischen, ein Stickstoffatom und gegebenenfalls als weiteres Heteroatom ein Sauerstoff-, Stickstoff-oder Schwefelatom enthaltenden 5-oder 6-Ring;

$R_3$, $R_4$ und $R_5$, die gleich oder verschieden sein können, Wasserstoff oder einen Alkylrest mit 1 - 4 Kohlenstoffatomen; $R_7$ und $R_8$, die gleich oder verschieden sein können, Hydroxy; Alkoxy mit 1 - 4 Kohlenstoffatomen; oder Alkylthio mit 1 - 4 Kohlenstoffatomen und

$R_6$ und $R_9$, die gleich oder verschieden sein können, Wasserstoff; Hydroxy; Alkoxy mit 1 - 4 Kohlenstoffatomen; Alkylthio mit 1 - 4 Kohlenstoffatomen; oder den Rest

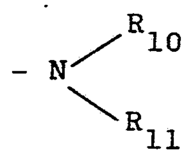

in dem

$R_{10}$ Wasserstoff; oder Alkyl mit 1 - 4 Kohlenstoffatomen und $R_{11}$ Wasserstoff; oder Alkyl mit 1 - 4 Kohlenstoffatomen, wobei der Alkylrest gegebenenfalls durch Hydroxy, Methoxy oder Furfuryl substituiert sein kann.

Bevorzugt sind solche Verbindungen der allgemeinen Formel I, in denen $R_1$ und $R_2$, die gleich oder verschieden sein können für Wasserstoff; eine geradkettige oder verzweigte Alkylgruppe mit 1 - 5 Kohlenstoffatomen oder $R_1$ für Wasserstoff und $R_2$ für Amino; Methylamino; Dimethylamino; Isopropylidenamino; Dimethylamino-$C_1$-$C_4$-alkyl; Methoxy-$C_1$-$C_4$-alkyl; Cyclopropyl; Cyclopentyl; Cyclohexyl; Cyclohexylmethyl; Phenyl; Phenylethyl, wobei der Phenylring gegebenenfalls ein-oder zweifach durch Methoxy oder Halogen substituiert ist; Pyrazolyl; Propargyl; [1-Methylpyrrolidin-2-yl]-ethyl; (Piperidin-1-yl)ethyl; Allyl; 4-Benzylpiperazin-1-yl; (Furan-2-yl)methyl; (Pyrrolidin-1-yl)-ethyl; 2-Hydroxyethyl; (Pyridin-4-yl)-ethyl; Benzyl; (Thiophen-3-yl) ethyl; oder $R_1$ und $R_2$ zusammen mit dem Stickstoffatom an welches sie gebunden sind für Pyrrolidin; Morpholin; Piperazin welches gewünschtenfalls durch Phenethyl oder Methoxyphenyl substituiert ist; stehen

und

$R_7$ und $R_8$ unabhängig voneinander für Wasserstoff; Methyl; Methoxy; Hydroxy; oder Methylthio

und

$R_3$, $R_4$, $R_5$, $R_6$ und $R_9$ für Wasserstoff stehen.

Die Verbindungen der Formel I sind Basen und können auf übliche Weise mit anorganischen oder organischen Säuren sowie Salz-und Komplexbildnern in beliebige physiologisch unbedenkliche Addukte (Salze) überführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Apfelsäure, Benzoesäure, p-Hydroxybenzoesäure, Phthalsäure, Zimtsäure, Salicylsäure, Ascorbinsäure, Methansulfonsäure und dergleichen.

Als Verbindungen der Formel I sind namentlich zu nennen:

3,4-Dihydro-6,7-dimethoxy-12-N,N-dimethylaminoethylamino-pyridazino [4',5' : 3,4] pyrrolo [2,1-a] isochinolin;

3,4-Dihydro-6,7-dimethoxy-12-hydrazino-pyridazino-[4',5' : 3,4] pyrrolo [2,1-a] isochinolin;

3,4-Dihydro-6,7-dimethoxy-12-dimethylamino-pyridazino-[4',5' : 3,4] pyrrolo [2,1-a] isochinolin;

3,4-Dihydro-6,7-dimethoxy-12-morpholino-pyridazino-[4',5' : 3,4] pyrrolo [2,1-a] isochinolin;

3,4-Dihydro-6,7-dimethoxy-pyridazino[4',5' : 3,4] pyrrolo-[2,1-a] isochinolin-12-acetonylhydrazon;

3,4-Dihydro-6,7-dimethoxy-12-anilino-pyridazino-[4',5' : 3,4] isochinolin;

3,4-Dihydro-6,7-dimethoxy-12-methylamino-pyridazino-[4',5' : 3,4] pyrrolo [2,1-a] isochinolin;

3,4-Dihydro-6,7-dimethoxy-12-ethylamino-pyridazino-[4',5' : 3,4] pyrrolo [2,1-a] isochinolin;

3,4-Dihydro-6,7-dimethoxy-12-diethylamino-pyridazino-[4',5' : 3,4] pyrrolo [2,1-a] isochinolin;

3,4-Dihydro-6,7-dimethoxy-12-cyclopropylamino-pyridazino-[4',5' : 3,4] pyrrolo [2,1-a] isochinolin;

3,4-Dihydro-6,7-dimethoxy-12-isopropylamino-pyridazino-[4',5' : 3,4] pyrrolo [2,1-a] isochinolin;

3,4-Dihydro-6,7-dimethoxy-12-butylamino-pyridazino-[4',5' : 3,4] pyrrolo [2,1-a] isochinolin;

3,4-Dihydro-6,7-dimethoxy-12-N,N-dimethylhydrazino-pyridazino [4',5' : 3,4] pyrrolo [2,1-a] isochinolin;

3,4-Dihydro-6,7-dimethoxy-12-(2-methoxyethyl)-amino-pyridazino [4',5' : 3,4] pyrrolo [2,1-a] isochinolin;

3,4-Dihydro-6,7-dimethoxy-12-[2-(3,4-dimethoxyphenyl)-ethyl]-amino-pyridazino [4',5' : 3,4] pyrrolo [2,1-a] isochinolin;

3,4-Dihydro-6,7-dimethoxy-12-(pyrazol-3-yl-amino)-pyridazino-[4',5' : 3,4] pyrrolo [2,1-a] isochinlin;

3,4-Dihydro-6,7-dimethoxy-12-(3-aminopyrazol-2-yl)-[4',5' : 3,4] pyrrolo [2,1-a] isochinolin

3,4-Dihydro-6,7-dimethoxy-12-cyclopentylamino-pyridazino-[4',5' : 3,4] pyrrolo [2,1-a] isochinolin;

3,4-Dihydro-6,7-dimethoxy-12-cyclohexylmethylaminopyridazino [4',5' : 3,4] pyrrolo [2,1-a] isochinolin

3,4-Dihydro-6,7-dimethoxy-12-propargylamino-pyridazino-[4',5' : 3,4] pyrrolo [2,1-a] isochinolin;

3,4-Dihydro-6,7-dimethoxy-1-2-[2-(1-methyl)-pyrrolidinyl]-ethyl -amino-pyridazino[4',5' : 3,4] pyrrolo [2,1-a] isochinolin;

3,4-Dihydro-6,7-dimethoxy-12-[2-(1-piperidinyl)-ethyl]-amino-pyridazino[4',5' : 3,4]pyrrolo [2,1-a]isochinolin;

3,4-Dihydro-6,7-dimethoxy-12-pyrrolidinyl-pyridazino-[4',5' : 3,4] pyrrolo [2,1-a]isochinolin;
3,4-Dihydro-6,7-dimethoxy-12-n-propylamino-pyridazino-[4',5' : 3,4] pyrrolo [2,1-a]isochinolin;
12-Allylamino-3,4-dihydro-6,7-dimethoxy-pyridazino-[4',5' : 3,4] pyrrolo[2,1-a]isochinolin;
3,4-Dihydro-6,7-dimethoxy-12-[4-(2-methoxyphenyl)-piperazinyl] pyridazino [4',5' : 3,4]pyrrolo[2,1-a]-isochinolin;
3,4-Dihydro-6,7-dimethoxy-12-(3-dimethylaminopropyl)-aminopyridazino [4',5' : 3,4]pyrrolo[2,1-a]isochinolin;
3,4-Dihydro-6,7-dimethoxy-12[2-(1-morpholinyl)-ethyl]-amino-pyridazino [4',5' : 3,4]pyrrolo[2,1-a]isochinolin;
3,4-Dihydro-6,7-dimethoxy-12-n-pentylamino-pyridazino-[4',5' : 3,4] pyrrolo [2,1-a]isochinolin;
3,4-Dihydro-6,7-dimethoxy-12-cyclohexylamino-pyridazino-[4',5' : 3,4] pyrrolo[2,1-a]isochinolin;
3,4-Dihydro-6,7-dimethoxy-12-[4-(2-phenylethyl)-piperazinyl]-pyridazino[4',5' : 3,4]pyrrolo[2,1-a]isochinolin;
3,4-Dihydro-6,7-dimethoxy-12-furfurylamino-pyridazino-[4',5' : 3,4] pyrrolo[2,1-a]isochinolin;
3,4-Dihydro-6,7-dimethoxy-12-(3-methoxypropylamino)-pyridazino[4',5' : 3,4]pyrrolo[2,1-a]isochinolin;
3,4-Dihydro-6,7-dimethoxy-12-isopentylamino-pyridazino-[4',5' : 3,4] pyrrolo[2,1-a]isochinolin;
3,4-Dihydro-6,7-dimethoxy-12-[2-(1-pyrrolidinyl)-ethyl]-amino-pyridazino[4',5' : 3,4]pyrrolo[2,1-a]isochinolin;
3,4-Dihydro-6,7-dimethoxy-12-(2-hydroxyethyl)-amino-pyridazino[4',5' : 3,4]pyrrolo[2,1-a]isochinolin;
3,4-Dihydro-6,7-dimethoxy-12-(4-fluoranilino)-pyridazino-[4',5' : 3,4] pyrrolo[2,1-a]isochinolin;
3,4-Dihydro-6,7-dimethoxy-12-[2-(4-pyridinyl)-ethyl]-amino-pyridazino [4',5' : 3,4]pyrrolo[2,1-a]isochinolin;
3,4-Dihydro-6,7-dimethoxy-12-[2-(2-pyridinyl)-ethyl)-aminopyridazino [4',5' : 3,4]pyrrolo[2,1-a]isochinolin;
12-(4-Benzylpiperazin-1-yl)amino-3,4-dihydro-6,7-dimethoxy-pyridazino [4',5' : 3,4]pyrrolo[2,1-a]isochinolin;
3,4-Dihydro-6,7-dimethoxy-12-[2-(3-thiophen-3-yl)-ethyl]-amino-pyridazino[4',5' : 3,4]pyrrolo[2,1-a]isochinolin;
3,4-Dihydro-6,7-dimethoxy-12-[2-(1-methyl)-pyrrolyl]-ethyl]-amino-pyridazino[4',5' : 3,4]pyrrolo[2,1-a]-isochinolin;
3,4-Dihydro-6,7-dimethoxy-12-[2-amino-pyridazino[4',5' : 3,4]-pyrrolo [2,1-a]isochinolin;
3,4-Dihydro-6,7-dimethoxy-12-[4-(2-phenylethyl)-piperazinyl]-pyridazino-[4',5' : 3,4]pyrrolo[2,1-a]isochinolin;
3,4-Dihydro-6,7-dimethoxy-12-isobutylamino-pyridazino[4',5' : 3,4] pyrrolo[2,1-a]isochinolin;
3,4-Dihydro-6,7-dimethoxy-12-benzylamino-pyridazino[4',5' : 3,4] pyrrolo[2,1-a]isochinolin;

Besonders hervorzuheben sind:

3,4-Dihydro-6,7-dimethoxy-12-(3-dimethylaminopropylamino)-pyridazino [4',5' : 3,4] pyrrolo [2,1-a]isochinolin
3,4-Dihydro-6,7-dimethoxy-12-[2-(1-methylpyrrolidin-2-yl)ethyl]amino-pyridazino [4',5' : 3,4] pyrrolo [2,1-a]-isochinolin
3,4-Dihydro-6,7-dimethoxy-12-[2-(pyrrolidin-1-yl)ethyl]amino-Pyridazino[4',5' : 3,4] pyrrolo [2,1-a]isochinolin und
3,4-Dihydro-6,7-dimethoxy-12-furfuryl-amino-pyridazino [4',5' : 3,4] pyrrolo [2,1-a] isochinolin

Aus den deutschen Patentanmeldungen DE 35 00 941.1 und DE 35 25 048.8 sind cardiotonisch wirksame Verbindungen der allgemeinen Formel I bekannt geworden.

Es wurde nun überraschend gefunden, daß die dort genannten Substanzen sowohl als freie Basen, wie auch in Form ihrer Salze mit Säuren, Basen oder Komplexbildern eine hervorragende cardio-und neuroprotektive Wirkung besitzen. Darüber hinaus fördern sie die Gewebsdurchblutung und Gewebssauerstoffversorgung im zentralen Nervensystem.

Versuchsergebnisse für Verbindungen A bis G werden angegeben:

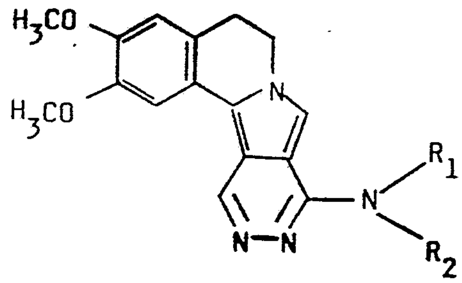 .HCl

| Verbindung | 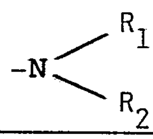 |
|---|---|
| A | $-NH-CH_2-CH_2-OCH_3$ |
| B | $-NHC_2H_5$ |
| C | $-NH-CH_2-$ 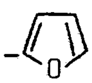 |
| D | $-NH-CH_2-CH=CH_2$ |
| E | $-NH-(CH_2)_3-N(CH_3)_2$ |
| F | $-NH-CH_2-CH_2-$ 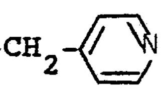 |
| G | $-NH-CH_2-CH_2-$ 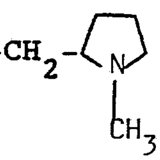 |

| Verbindung | 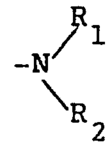 |
|---|---|
| H | $-NH-CH_2-C_6H_5$ |
| I* | 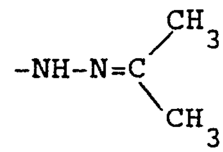 |
| J | 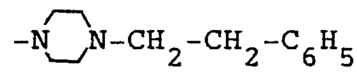 |
| K | 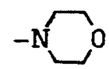 |
| L | $-NH-CH(CH_3)_2$ |
| M | 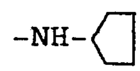 |
| N* | 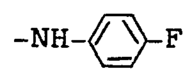 |
| O | 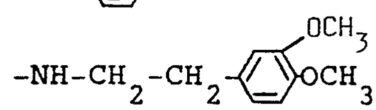 |
| P | 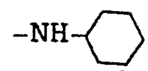 |
| Q | 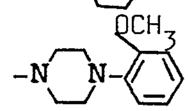 |

*Base

Die cardioprotektive Wirkung wird auf folgende Weise bestimmt:
Bekanntlich ist der mycardiale $Ca^{+2}$-Gehalt ein Maß für die hypoxische bzw. die durch toxische Catecholamindosen hervorgerufene Herzschädigung (Higgins et al., Mol. Cell. Cardiol. 10: 427 - 438, 1984; Nakanishi et al., Am. J. Physiol. 242: 437 - 449, 1982; Fleckenstein, A., Vorträge der Erlanger Physiol. Tagung 1970, Ed. Keidel, Springer Verlag Berlin, Heidelberg, New York, 1971). Umgekehrt ist die Inhibition der hypoxischen oder Isoprenalin-bedingten myocardialen Calciumaufnahme ein Maß für die cardioprotektive Effektivität von Calciumantagonisten (Fleckenstein s.o.), von Calmodulininhibitoren (Higgins) und anderen Pharmaka, z.B. Betaadrenolytika (Arndts, Arzneimittelforschung 25: 1279 -1284, 1975).

Die cardioprotektive Wirkung wurde an wachen Ratten nach subcutaner oder peroraler Wirkstoffgabe anhand der von Arndts (s.o.) beschriebenen Methode festgestellt, und die Wirkungsstärke der Testsubstanzen als $H_{50}$-Wert angegeben; dieser Wert entspricht der Dosis, die die durch eine Gabe von 30 (mg/kg; s.c.) Isoprenalin bedingte, myocardiale Radio-Calciumaufnahme zu 50 % hemmt. Hier erwiesen sich die untersuchten neuen Verbindungen als ebenso wirksam wie Nifedipin. .

| Verbindung | $H_{50}$-Wert |
|---|---|
| A | 1,92 |
| C | 0,82 |
| D | 2,42 |
| E | 0,46 |
| F | 2,14 |
| G | 0,38 |

Bestimmung der Calcium-Ionenkinetik an Kaninchen-Aortenstreifen

Methode nach C. van Breemen, P. Aaronson, R. Loutzenheiser und K. Meisheri
CHEST 78: 157s - 165s (1980)
und R. Casteels & G. Droogman
J. Physiol 317: 263 - 279 (1981)

Methode: Mit $^{45}Ca^{2+}$ geladene Aortenstreifen werden sequentiell äquilibriert in physiologischer Lösung. Dabei tauscht das intrazelluläre $Ca^{2+}$ mit dem extrazellulären $Ca^{2+}$ aus, das heißt im extrazellulären Bad ist das radioaktive $^{45}Ca^{2+}$ meßbar. Auf eine Stimulation mit 80 mM $K^+$ oder $10^{-5}$ Mol/l Noradrenalin (NA) hin wird der Austausch beschleunigt. Dabei ist die im Bad ansteigende Radioaktivität ein Maß für den $Ca^{2+}$-Efflux. So wird das Ausmaß der durch die Stimulation freigesetzten $Ca^{2+}$ als Effluxrate bestimmt.

Ergebnis: Die Substanzen B, C und E potenzieren den durch 80 mM $K^+$ stimulierten $Ca^{2+}$ -Efflux entsprechend um 27, 77 und 11 %. Damit ist eine calciumantagonistische Wirkung ausgeschlossen. Dagegen wird der mit NA stimulierte $CA^{2+}$-Efflux um entsprechend 12,26 und 19 % gehemmt. Das läßt auf eine schwache Hemmung einer Überstimulation schließen. Beide gemessene Eigenschaften zeigen einen Effekt der Substanzen auf die intrazelluläre $Ca^{2+}$ -Konzentration: Die intrazelluläre $Ca^{2+}$ -Versorgung der Zellen wird verbessert und bis zu einem gewissen Grad vor Übererregung geschützt.

Diese Ergebnisse zeigen deutlich die zellprotektive Wirkung, die speziell für Herz-und Nervenzellen zutrifft.

Die Untersuchungen am isolierten Langendorff-Herzen wurden nach O. Langendorff, Pflügers Arch. 61, 291 (1895) mit kleinen Abwandlungen ausgeführt:

Isoliertes Rattenherz, retrograde Perfusion über die Aorta. Elektrische Stimulation (300 Impulse/min. Impulsdauer lmsec.). Messung des linksventrikulär entwickelten Druckes mit flüssigkeitsgefülltem Latex-Ballon. Nach 20 min Aequilibrierung Auslösen eines Sauerstoff-Mangelzustandes mit 100-fach vermindertem Fluß durch Drosselung des Perfusionsflusses für 60 min, Rückkehr zu normalem Fluß bewirkt starken Anstieg der Grundspannung, unter Kontrollbedingungen setzt die Herztätigkeit nach 6 - 7 min unregelmäßig wieder ein, nach 15 -18 min rascher Übergang zu regelmäßigen Kontraktionen. Cardioprotektiv wirksame Verbindungen verkürzen die Zeit bis zum Einsetzen regelmäßiger Kontraktionen. Infusion der Testsubstanz (gelöst in unbegastem Perfusionsmedium) während des $0_2$-Mangelzustandes herznah in das Perfusionssystem.

Die getesteten Verbindungen ergaben bei Anwendung in der unten angegebenen Konzentration eine signifikante Reduktion der Zeit bis zum Einsetzen regelmäßiger Kontraktionen.

| Verbindung | Konzentration |
|---|---|
| C | 6,6 µg/ml |
| E | 13,3 µg/ml |

Die neuroprotektive Wirkung der erfindungsgemäßen Verbindungen kann anhand der folgenden Untersuchung gezeigt werden.

In Vesuchen mit Einschränkung des Kurzzeitgedächtnisses bzw. Behinderung des Übergangs von Inhalten des Kurzzeit-in das Langzeitgedächtnis durch Gabe des muskarin-cholinergen Antagonisten Scopolamin (0,6 mg/kg; i.p.); (Psychopharmacology 78, S. 104-111 (1982)) sind die Verbindungen in der Lage, dieser pharmakologisch induzierten cerebralen Insuffizienz entgegenzuwirken bzw. sie aufzuheben.

Bei der Prüfung der Überlebensfähigkeit von Tieren in einer geschlossenen Kammer (HYPOXIE-TOLERANZ-TEST) welche mit einem Gasgemisch, bestehend aus 96,5 % Stickstoff und 3,5 % Sauerstoff, durchströmt wurde, wiesen die mit den neuen Substanzen vorbehandelten Tiere eine statistisch signifikant höhere Überlebensfähigkeit auf als Kontrolltiere bzw. mit Diltiazem, Verapamil oder Nifedipin vorbehandelte Tiere.

Die mit dieser Methode geprüfte hirnprotektive Wirkung (Neuroprotektion) der Substanzen war bereits bei einer Dosis von 1 mg/kg p.o. ausgeprägt. Damit sind die neuen Verbindungen sowohl hinsichtlich der wirksamen Dosis als auch der tierexperimentell erzielten Leistungsverbesserung den genannten bekannten Substanzen deutlich überlegen.

Die Mikrointophorese wurde gemäß Standardmethode durchgeführt.

| Verbindung | Hypoxie | Scopolamin-Demenz | Mikroiontophorese |
|---|---|---|---|
| H | | wirksam | |
| I | wirksam | | |
| J | wirksam | | |
| K | | wirksam | |
| L | wirksam | | |
| M | wirksam | | |
| N | wirksam | | |
| O | | wirksam | |
| P | | wirksam | |
| B | wirksam | | |
| Q | wirksam | | |
| E | wirksam | | wirksam |
| G | wirksam | | wirksam |
| Diltiazem | unwirksam | unwirksam | |
| Verapamil | schwach w. | unwirksam | |
| Nifedipin | unwirksam | 1 mg/kg wirksam<br>3-30 unwirksam | |

wirksam: Im Dosenbereich bis einschließlich 30 mg/kg zeigen in der mit Substanz behandelten Tiergruppe signifikant mehr Tiere den gewünschten Effekt als in der Kontrollgruppe.

Aus den Untersuchungsergebnissen kann geschlossen werden, daß die hier beschriebenen Verbindungen cardio-und/oder neuroprotektive Wirkung haben und daher als Wirkstoffe für Arzneimittel gegen zum Beispiel cerebrale Stoffwechselstörungen bzw. das hirnorganische Psychosyndrom, sowie die posttraumatische und alkoholische Hirnschädigung Verwendung finden. Sie können bei der Behandlung von Krankheitszuständen verwendet werden, bei denen Veränderungen auftreten wie beispielsweise cholinerges Defizit

(z.B. M. Alzheimer, senile Demenz, anticholinerges Syndrom), bei cognitiven Störungen (zum Beispiel im Alter), Mangeldurchblutung im Gehirn (z.B. verursacht durch Arterioschlerose oder Schlaganfall). Ferner sind sie geeignet für die Behandlung der koronaren Herzerkrankung und des akuten Myokardinfarkts und können auch zur Propylaxe angewendet werden.

Die Verbindungen können sowohl enteral als auch parenteral verabreicht werden. Als Dosis für die orale Anwendung werden 0,1 bis 500 mg Wirkstoff pro Dosis, für die i.v.-Anwendung von 0,05 bis 150 mg pro Dosis vorgeschlagen. Der gewünschte therapeutische Dosis ist von der Indikation und Darreichungsform abhängig und kann experimentell bestimmt werden.

Geeignete Anwendungsformen sind beispielsweise Tabletten, Kapseln, Zäpfchen, Lösungen, Säfte, Emuslionen, Aerosole oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageehüllen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure, hergestellt und in Injektionsflaschen oder Ampullen abgefüllt.

Die eine oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol bzw. dessen Derivaten herstellen.

Verfahen zur Herstellung der Verbindungen der Formel I sind in den deutschen Patentanmeldungen DE 35 00 941.1 und DE 35 25 048.8 beschrieben auf die hiermit Bezug genommen wird.

## Ansprüche

1) Verwendung von Verbindungen der Formel (I)

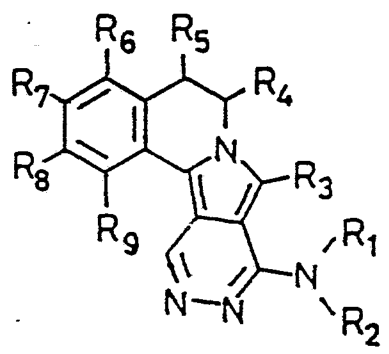

(I)

in der

$R_1$ und $R_2$, die gleich oder verschieden sein können, Wasserstoff; $C_3$-$C_7$ Cycloalkyl; $C_2$-$C_5$ Alkenyl; Phenyl (wobei der Phenylring gegebenenfalls ein-oder zweifach durch Halogen oder Methoxy substituiert ist);

Propargyl; einen geradkettigen oder verzweigten, gesättigten oder ungesättigten Alkylrest mit 1 - 5 Kohlenstoffatomen, der substituiert sein kann durch Hydroxy, Alkoxy mit 1 - 4 Kohlenstoffatomen, Halogen, $NH_2$, NH-Alkyl mit 1 - 2 Kohlenstoffatomen, N,N-Di($C_1$-$C_2$)alkylamino, NH-Acyl mit 2 - 4 Kohlenstoffatomen, Cycloalkyl mit 3 - 7 Kohlenstoffatomen, Phenyl (wobei der Phenylring seinerseits substituiert sein kann, ein-oder mehrfach durch Halogen, Alkyl mit 1 - 2 Kohlenstoffatomen, Alkoxy mit 1 - 2 Kohlenstoffatomen, NH-Alkyl mit 1 - 2 Kohlenstoffatomen, N,N-Dialkyl mit 1 - 2 Kohlenstoffatomen, $NH_2$, N-Acyl mit 2 - 3 Kohlenstoffatomen oder Alkylsulfonylamino), Furyl, Thienyl, einen stickstoffhaltigen heterocyclischen 5-oder 6-Ring der gegebenenfalls als weiteres Heteroatom ein Sauerstoff-oder Schwefelatom enthalten kann (wobei der Ring gegebenenfalls durch Alkyl mit 1 - 4 Kohlenstoffatome substituiert ist); bedeuten; oder

$R_1$ und $R_2$ zusammen mit dem Stickstoffatom einen 3-bis 7-gliedrigen Ring, der gegebenenfalls als weiteres Heteroatom ein Sauerstoff-oder ein Stickstoffatom enthalten kann (wobei der Ring gegebenenfalls durch Phenyl-($C_1$-$C_4$)-Alkyl substituiert ist (wobei der Phenylring seinerseits ein-oder zweifach durch Halogen oder Methoxy substituiert ist)); bedeuten; oder

$R_2$, falls $R_1$ Wasserstoff bedeutet, auch $-NH_2$; Di($C_1$-$C_2$) Alkylamino; Acetonylamino; -NH($C_2$-$C_3$)Acyl; einen Alkylsulfonyl-oder Alkoxycarbonylrest mit je 1 - 3 Kohlenstoffatomen in der Alkylkette; die Isopropylidenaminogruppe

( 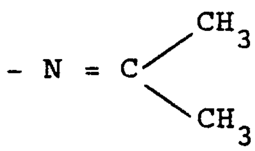 )

oder einen heterocyclischen, ein Stickstoffatom und gegebenenfalls als weiteres Heteroatom ein Sauerstoff-, Stickstoff-oder Schwefelatom enthaltenden 5-oder 6-Ring bedeuten kann;

$R_3$, $R_4$ und $R_5$, die gleich oder verschieden sein können, Wasserstoff oder einen Alkylrest mit 1 - 4 Kohlenstoffatomen bedeuten;

$R_7$ und $R_8$, die gleich oder verschieden sein können, Hydroxy; Alkoxy mit 1 - 4 Kohlenstoffatomen; oder Alkylthio mit 1 - 4 Kohlenstoffatomen bedeuten und

$R_6$ und $R_9$, die gleich oder verschieden sein können, Wasserstoff; Hydroxy; Alkoxy mit 1 - 4 Kohlenstoffatomen; Alkylthio mit 1 - 4 Kohlenstoffatomen; oder den Rest

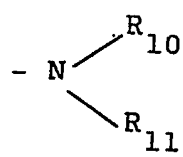

bedeuten, in dem

$R_{10}$ Wasserstoff; oder Alkyl mit 1 - 4 Kohlenstoffatomen ist und

$R_{11}$ Wasserstoff; oder Alkyl mit 1 - 4 Kohlenstoffatomen, wobei der Alkylrest gegebenenfalls durch Hydroxy, Methoxy oder Furfuryl substituiert sein kann; sowie deren physiologisch verträglichen Salze mit Säuren, Basen oder Komplexbildnern zur Herstellung von Mitteln zur Cardioprotektion und/oder zur Neuroprotektion.

2) Verwendung von Verbindungen der Formel I nach Anspruch 1 in der $R_1$ und $R_2$, die gleich oder verschieden sein können für Wasserstoff; oder eine geradkettige oder verzweigte Alkylgruppe mit 1 - 5 Kohlenstoffatomen oder $R_1$ für Wasserstoff und $R_2$ für Amino; Methylamino; Dimethylamino; Isopropylidenamino; Dimethylamino-$C_1$-$C_4$-alkyl; Methoxy-$C_1$-$C_4$-alkyl; Cyclopropyl; Cyclopentyl; Cyclohexyl; Cyclohexylmethyl; Phenyl; Phenylethyl, wobei der Phenylring gegebenenfalls ein-oder zweifach durch Methoxy oder Halogen substituiert ist; Pyrazolyl; Propargyl; [1-Methylpyrrolidin-2-yl]-ethyl; (Piperidin-1-yl)ethyl; Allyl; 4-Benzyl-piperazin-1-yl; (Furan-2-yl)methyl; (Pyrrolidin-1-yl)-ethyl; 2-Hydroxyethyl; (Pyridin-4-yl)-ethyl; Benzyl; (Thiophen-3-yl) ethyl; oder $R_1$ und $R_2$ zusammen mit dem Stickstoffatom an welches sie gebunden für Pyrrolidin; Morpholin; Piperazin welches gewünschtenfalls durch Phenethyl oder Methoxyphenyl substituiert ist; stehen

und

$R_7$ und $R_8$ unabhängig voneinander für Wasserstoff; Methyl; Methoxy; Hydroxy; oder Methylthio

und

$R_3$, $R_4$, $R_5$, $R_6$ und $R_9$ für Wasserstoff stehen.

3) Verwendung von
3,4-Dihydro-6,7-dimethoxy-12-[(3-d(methylaminopropyl)amino])-pyridazino [4',5' : 3,4] pyrrolo [2,1-a]-isochinolin
3,4-Dihydro-6,7-dimethoxy-12-[2-(1-methylpyrrolidin-2-yl)ethyl] amino-pyridazino [4',5' : 3,4] pyrrolo [2,1-a]-isochinolin
3,4-Dihydro-6,7-dimethoxy-12-[2-(pyrrolidin-1-yl)ethyl]amino-Pyridazino [4',5' : 3,4] pyrrolo [2,1-a]isochinolin und
3,4-Dihydro-6,7-dimethoxy-12-furfuryl-amino-pyridazino [4',5' : 3,4] pyrrolo [2,1-a] isochinolin sowie deren physiologisch verträglichen Salze mit Säuren und Basen zur Herstellung von Mitteln zur Cardioprotektion und/oder Neuroprotektion.